# DEMANDE DE BREVET EUROPEEN

(11) **EP 1 738 740 A2**
(43) Date de publication de la demande: **03.01.2007**
(21) Numéro de dépôt: 06115254.2
(22) Date de dépôt: 09.06.2006
(51) Int. Cl.: A61K 8/49, A61K 31/426, A61Q 5/10, A61Q 19/04

(54) **Utilisation de composés benzyl-1,3-thiazolidine-2,4-diones pour favoriser et/ou induire et/ou stimuler la pigmentation des matières kératiniques et/ou limiter leur dépigmentation et/ou leur blanchiment.**

(30) Priorité: 28.06.2005 FR 0551787
(71) Demandeur: L'Oreal, 75008 Paris (FR)
(72) Inventeur: Boulle, Christophe, 77400 Lagny S/Marne (FR); Dalko, Maria, 91190 Gif S/Yvette (FR)
(74) Mandataire: Miszputen, Laurent

(57) **Abrégé**

La présente invention concerne l'utilisation cosmétique d'au moins un composé benzyl-1,3-thiazolidine-2,4-dione de formule (I) suivante ou d'un de ses sels et/ou de ses solvates : 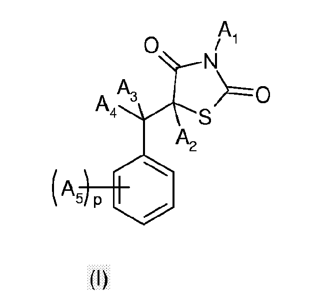 comme agent pour favoriser et/ou induire et/ou stimuler la pigmentation des matières kératiniques et/ou comme agent pour prévenir et/ou limiter la dépigmentation et/ou le blanchiment des matières kératiniques et plus particulièrement des fibres kératiniques humaines comme les cheveux, les poils de barbe, les poils de moustache, les cils et les sourcils.

La présente invention concerne plus particulièrement l'utilisation cosmétique d'au moins un composé benzyl-1,3-thiazolidine-2,4-dione de formule (I) ou d'un de ses sels et/ou de ses solvates comme agent pour prévenir et/ou limiter la canitie desdites fibres kératiniques humaines.

## Description

L'invention a pour objet l'utilisation en cosmétique d'au moins un composé benzyl-1,3-thiazolidine-2,4-dione de formule particulière comme agent pour favoriser et/ou induire et/ou stimuler la pigmentation des matières kératiniques et/ou limiter leur dépigmentation et/ou leur blanchiment et plus particulièrement comme agent pour prévenir et/ou limiter la canitie des fibres kératiniques humaines.

Les matières kératiniques auxquelles s'applique l'invention comprennent la peau, les ongles humains et les fibres kératiniques humaines telles que les cheveux, les sourcils, les cils, les poils de barbe, de moustache. L'invention s'applique aussi aux matières kératiniques des mammifères de l'espèce animale (chien, cheval ou chat par exemple). Plus spécialement, l'invention s'applique aux cheveux, aux poils de barbe, aux poils de moustache, aux cils et aux sourcils humains.

Il existe un besoin de nouveaux produits de soin et/ou de traitement des matières kératiniques humaines favorisant leur pigmentation et/ou limitant leur dépigmentation et plus particulièrement des produits permettant de prévenir et/ou diminuer la canitie des fibres kératiniques humaines telles que les cheveux, les cils et/ou certains poils.

La couleur des cheveux et de la peau humaine est fonction de différents facteurs et notamment des saisons de l'année, de la race, du sexe et de l'âge. Elle est principalement déterminée par la concentration en mélanine produite par les mélanocytes. Ces derniers sont des cellules spécialisées qui par l'intermédiaire d'organelles particuliers, les mélanosomes, synthétisent la mélanine.

La synthèse de la mélanine (ou mélanogénèse) est complexe et fait intervenir schématiquement les principales étapes suivantes :

Tyrosine ---> Dopa ---> Dopaquinone ---> Dopachrome ---> Mélanine

La tyrosinase (monophénol dihydroxyl phénylalanine : oxygen oxydoreductase EC 1.14.18.1) intervient dans cette suite de réactions en catalysant notamment la réaction de transformation de la tyrosine en Dopa (dihydroxyphénylalanine) et la réaction de transformation de la Dopa en Dopaquinone.

La partie supérieure du follicule pileux se présente comme une invagination tubulaire de l'épiderme qui s'enfonce jusqu'aux couches profondes du derme. La partie inférieure, ou bulbe pileux, comporte elle-même une invagination dans laquelle se trouve la papille dermique. On trouve, autour de la papille dermique, dans la partie inférieure du bulbe une zone peuplée de cellules à haut taux de prolifération (cellules de la matrice). Ces cellules sont les précurseurs des cellules kératinisées qui constitueront le cheveu. Les cellules qui résultent de la prolifération de ces précurseurs migrent verticalement dans le bulbe et se kératinisent progressivement dans la partie supérieure du bulbe ; cet ensemble de cellules kératinisées formera la tige pilaire. La pigmentation du cheveu et des poils requiert la présence de mélanocytes au niveau du bulbe du follicule pileux. Ces mélanocytes sont dans un état actif, c'est-à-dire qu'ils synthétisent des mélanines (ou pigments mélaniques). Ces pigments sont transmis aux kératinocytes destinés à former la tige pilaire ce qui conduira à la pousse d'un cheveu ou d'un poil pigmenté. Cette structure est appelée "unité folliculaire de pigmentation".

On sait que dans la plupart des populations, la coloration brune de la peau et le maintien d'une coloration constante du cheveu sont des aspirations importantes.

Il est admis que l'apparition de poils et/ou de cheveux gris ou blancs, ou canitie, est associée à une diminution de mélanine dans la tige pilaire. Ce phénomène survient naturellement au cours de la vie d'un individu. Toutefois, l'être humain cherche à avoir un aspect plus jeune et dans un but esthétique, il est souvent tenté de lutter contre ce phénomène, surtout lorsqu'il se produit à un âge relativement précoce.

On a ainsi proposé de nombreuses solutions dans le domaine de la coloration artificielle par apport de colorants exogènes visant à donner aux cheveux une coloration la plus proche possible de ce qu'elle possède naturellement. Une autre approche consiste à stimuler la voie naturelle de la pigmentation.

Parmi les solutions proposées, on peut citer des compositions contenant un inhibiteur de phosphodiestérases (WO 95/17161), des fragments d'ADN (WO 95/01773), du diacyl glycérol (WO 94/04122), des prostaglandines (WO 95/11003) ou des dérivés de pyrimidine 3-oxyde (EP 829260).

De manière inattendue, la demanderesse a maintenant trouvé qu'il était possible de stimuler la synthèse de mélanine par les mélanocytes en inhibant spécifiquement la dégradation des prostaglandines synthétisés par ces mélanocytes ou celles présentes dans son environnement.

L'implication de certaines prostaglandines dans la pigmentation des poils ou de la peau chez l'homme ou chez l'animal est décrite dans le document Wand M., 1997, Arch. Ophtalmol., 115 ; Abdel Malek et al., 1987, cancer Res., 47. Cependant, les prostaglandines étant des molécules au temps de demi-vie biologique très court et du fait du caractère local et labile de leur métabolisme (Narumiya S. et al.), il semble important de pouvoir prolonger l'activité des prostaglandines impliquées dans la pigmentation de la peau, des poils et/ou des cheveux humains.

Dans la demande de brevet WO 04/073594, la demanderesse avait montré que la 15 hydroxy prostaglandine déshydrogénase (15-PGDH) était également exprimée dans le mélanocyte du cheveu, ce qui n'avait jamais été mis en évidence jusqu'à présent. En outre, elle a mis en évidence la présence de 15-PGDH dans la papille dermique et le mélanocyte du cheveu et a proposé d'utiliser un inhibiteur de 15-PGDH pour favoriser la pigmentation de la peau, des poils et/ou des cheveux humains. Il est maintenant possible de réguler localement le taux de prostaglandines notamment celui présent au niveau du mélanocyte, en particulier du cheveu, en agissant sur la dégradation catalysée à la fois par la 15-PGDH du mélanocyte et du fibroblaste de la papille dermique.

La 15-PGDH de type 1 est une enzyme clé dans la désactivation des prostaglandines, en particulier de la PGF2-α et de la PGE2, qui sont des médiateurs importants de la croissance et la survie du cheveu. Elle répond à la classification EC 1.1.1.141 et est NAD+ dépendante. Cette enzyme catalyse une réaction d'oxydation sur le carbone 15 de l'hydroxyle en cétone. Elle a été isolée de rein de porc ; on a notamment observé son inhibition par une hormone thyroïdienne, la tri-iodo thyronine, à des doses très supérieures aux doses physiologiques. La 15-PGDH de type 2 est quant à elle NADP dépendante.

Dans cette même demande, la demanderesse avait, en outre, montré que les mélanocytes de cheveux exprimaient la prostaglandine H synthase 1 (PGHS-1 ou COX-1, E.C : 1.14.99.1). Ceci démontre pour la première fois que les mélanocytes de cheveux possèdent un métabolisme des prostaglandines, autonome.

Dans le document WO 04/073594, on a, de plus, montré qu'il était possible d'inhiber spécifiquement la 15-PGDH présente au niveau de la papille dermique et/ou du mélanocyte de cheveu. Une telle inhibition permet donc de freiner la désactivation des prostaglandines dans l'environnement du mélanocyte de cheveu. Les prostaglandines peuvent donc continuer par voie autocrine ou paracrine de stimuler les mélanocytes. En effet, l'application de tels inhibiteurs stimule la production par les mélanocytes de mélanine.

Selon l'invention, par « inhibiteur de la 15-PGDH », on entend toute substance, composé simple ou complexe, d'origine naturelle ou synthétique, capable d'inhiber ou de diminuer l'activité de l'enzyme 15-PGDH, et/ou capable d'inhiber, diminuer ou ralentir la réaction catalysée par cette enzyme. Les inhibiteurs de 15-PGDH selon l'invention sont de préférence des inhibiteurs de la 15-PGDH de type 1.

Avantageusement, l'inhibiteur est un inhibiteur spécifique de la 15-PGDH de type 1, NAD dépendante.

Aussi, de façon surprenante, la demanderesse a trouvé que certains composés benzyl-1,3-thiazolidine-2,4-diones de formule (I) que l'on définira en détail plus loin, étaient des inhibiteurs de la 15-hydroxy prostaglandine déshydrogénase en particulier de type 1. La demanderesse a par ailleurs trouvé que ces mêmes composés composés benzyl-1,3-thiazolidine-2,4-diones permettaient de favoriser et/ou induire et/ou stimuler la pigmentation des matières kératiniques ainsi que de limiter leur dépigmentation et/ou leur blanchiment.

La présente invention a donc pour objet l'utilisation cosmétique d'au moins un composé benzyl-1,3-thiazolidine-2,4-dione de formule (I) ou d'un de sels et/ou de ses solvates, comme agent pour favoriser et/ou induire et/ou stimuler la pigmentation des matières kératiniques et/ou comme agent pour prévenir et/ou limiter la dépigmentation et/ou le blanchiment des matières kératiniques et plus particulièrement des fibres kératiniques humaines comme les cheveux, les poils de barbe, les poils de moustache, les cils et les sourcils humains.

La présente invention concerne plus particulièrement l'utilisation cosmétique d'au moins un composé benzyl-1,3-thiazolidine-2,4-dione de formule (I) ou d'un de sels et/ou de ses solvates, comme agent pour prévenir et/ou limiter la canitie des fibres kératiniques humaines.

La présente invention a aussi pour objet l'utilisation cosmétique d'au moins un composé benzyl-1,3-thiazolidine-2,4-dione de formule (I) ou d'un de sels et/ou de ses solvates dans une composition de soin et/ou de maquillage pour induire et/ou stimuler la pigmentation des matières kératiniques et/ou limiter leur dépigmentation et/ou leur blanchiment et plus particulièrement des fibres kératiniques humaines comme les cheveux, les poils de barbe, les poils de moustache, les cils et les sourcils humains.

La présente invention concerne plus particulièrement l'utilisation cosmétique d'au moins un composé benzyl-1,3-thiazolidine-2,4-dione de formule (I) ou d'un de sels et/ou de ses solvates, dans une composition de soin et/ou de maquillage pour prévenir et/ou limiter la canitie des fibres kératiniques humaines.

La présente invention a aussi pour objet l'utilisation cosmétique d'au moins un composé benzyl-1,3-thiazolidine-2,4-dione de formule (I) ou d'un de sels et/ou de ses solvates dans la fabrication d'une composition destinée à induire et/ou stimuler la pigmentation des matières kératiniques et/ou limiter leur dépigmentation et/ou leur blanchiment et plus particulièrement des fibres kératiniques humaines comme les cheveux, les poils de barbe, les poils de moustache, les cils et les sourcils humains.

La présente invention concerne plus particulièrement l'utilisation d'au moins un composé benzyl-1,3-thiazolidine-2,4-dione de formule (I) ou d'un de sels et/ou de ses solvates, dans la fabrication d'une composition destinée à prévenir et/ou limiter la canitie des fibres kératiniques humaines comme les cheveux, les poils de barbe, les poils de moustache, les cils et les sourcils humains.

La présente invention concerne également un procédé cosmétique pour induire et/ou stimuler la pigmentation des matières kératiniques et plus particulièrement des fibres kératiniques humaines et/ou limiter leur dépigmentation et/ou leur blanchiment, caractérisé par le fait qu'il consiste à appliquer sur lesdites matières kératiniques, une quantité efficace d'au moins un composé benzyl-1,3-thiazolidine-2,4-dione de formule (I) ou d'un de sels et/ou de ses solvates.

La présente invention concerne également un procédé cosmétique pour traiter la canitie des fibres kératiniques humaines en particulier des cheveux, des poils de barbe, des poils de moustache, des cils et/ou des sourcils, caractérisé par le fait qu'il consiste à appliquer sur lesdites fibres, une quantité efficace d'au moins un composé benzyl-1,3-thiazolidine-2,4-dione de formule (I) ou d'un de sels et/ou de ses solvates.

Les composés benzyl-1,3-thiazolidine-2,4-dione conformes à l'invention répondent à la formule (I) suivantes ou correspondent à l'un de leurs sels et/ou de leurs solvates : 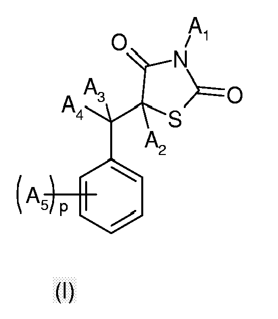
a) A₁ désigne :
   1) un atome d'hydrogène ;
   2) un groupe alkyle en C₁-C₂₀ linéaire ou ramifié, saturé ou insaturé éventuellement substitué par un ou plusieurs groupes Z₁ ;
b) A₂, A₃, A₄ et A₅ désignent indépendamment :
   1) un atome d'hydrogène ;
   2) un groupe Z₁ ;
c) p est compris entre 0 et 5 inclus et dans le cas où n>1, les substituants A₅ peuvent être identiques ou différents ;
d) Z₁ désigne
   1) un halogène comme F, CI, Br,
   2) un groupe choisi parmi CF₃, OCF₃, CN, NO₂, OZ₂, OCOZ₂, OCONZ₂Z'₂, SZ₂, SCOZ₂, SCONZ2Z'₂, SCSOZ₂, SCSNZ₂Z'₂, NZ₂Z'₂, NZ₂COZ'₂, NZ₂CONZ'₂Z''₂, NZ₂C(=NZ'₂)NZ''₂Z'''₂, NZ₂SO₂Z'₂, COZ₂, COOH, CONZ₂Z'₂, CSZ₂, CSNZ₂Z'₂ SO₃Z, SO₂NZ₂Z'₂, SO₂Z₂, SiZ₂Z'₂Z''₂, Si(OZ₂)(OZ'₂)OZ''₂ ;
   3) un groupe alkyle en C₁-C₂₀, linéaire ou ramifié, saturé ou insaturé,
e) les radicaux Z₂, Z'₂, Z''₂ et Z'''₂ indépendamment désignent :
   1) un atome d'hydrogène ;
   2) un groupe alkyle en C₁-C₂₀ linéaire ou ramifié, saturé ou insaturé, éventuellement substitués par un ou plusieurs groupes Z₃ ;
   3) un cycle de 4 à 15 atomes, saturés ou insaturés, contenant éventuellement au moins un hétéroatome parmi O, N, S, éventuellement accolé à un autre cycle, ces cycles étant éventuellement substitués par un ou plusieurs groupes Z₃. Ce cycle est avantageusement un phényle ;
f) le radical Z₃ représente :
   1) un groupe Z₄ ;
   2) un groupe alkyle en C₁-C₂₀ linéaire ou ramifié, saturé ou insaturé, éventuellement substitué par un ou plusieurs groupes Z₄ ;
   3) un cycle de 4 à 15 atomes, saturés ou insaturés, contenant éventuellement au moins un hétéroatome parmi O, N, S, éventuellement accolé à un autre cycle, ces cycles étant éventuellement substitués par un ou plusieurs groupes Z₄ ;
g) Z₄ représente :
   1) un halogène comme F, CI, Br,
   2) un des groupes CF₃, OCF₃, CN, NO₂, OZ₅, OCOZ₅, OCONZ₅Z'₅, SZ5, SCOZ₅, SCONZ₅Z'₅, SCSOZ₅, SCSNZ₅Z'₅, NZ₅Z'₅, NZ₅COZ'₅, NZ₅CONZ'₅Z"₅, NZ₅C(=NZ'₅)NZ"₅Z"'₅, NZ₅SO₅Z'₅, COZ₅, COOZ₅, CONZ₅Z'₅,CSZ₅, CSNZ₅Z'₅, SO₃Z, SO₂NZ₅Z'₅, SO₂Z5, SiZ₅Z'₅Z"₅, Si(OZ₅)(OZ'₅)OZ"₅;
h) les radicaux Z₅, Z'₅, Z"₅ et Z'''₅ indépendamment représentent
   1) un atome d'hydrogène
   2) un groupe alkyle en C₁-C₂₀ linéaire ou ramifié, saturé ou insaturé,
   3) un cycle de 4 à 15 atomes, saturés ou insaturés, contenant éventuellement au moins un hétéroatome parmi O, N, S, éventuellement accolé à un autre cycle, ces cycles étant éventuellement substitués par un ou plusieurs groupes CF₃, halogène, alkyle en C₁-C₂₀ linéaire ou ramifié, saturé ou insaturé.
   sous réserve que lorsque A₅ est situé en para du groupe CA₃A₄ :
   (i) lorsque Z₁ désigne OZ₂ ou COZ₂ alors Z₂ désigne un cycle tel que défini précédemment ;
   (ii) A₅ n'est pas un phényle ;
   (iii) lorsque Z₁ désigne NZ₂Z'₂ alors Z₂ désigne un groupe alkyle tel que défini précédemment
   comme agent pour induire et/ou stimuler la pigmentation des matières kératiniques et/ou limiter leur dépigmentation et/ou leur blanchiment.

Par sels de composé de formule (I), on entend selon l'invention, les sels organiques ou inorganiques d'un composé de formule (I).

Comme sels inorganiques utilisables selon l'invention on peut citer les sels de sodium ou de potassium ainsi que les sels d'ammonium, de zinc (Zn²⁺), de calcium (Ca²⁺), de cuivre (Cu²⁺), de fer (Fe²⁺ et Fe³⁺), de strontium (Sr²⁺), de magnésium (Mg²⁺) et de manganèse (Mn²⁺) ; les hydroxydes, les halogénohydrates (chlorhydrates par exemple), les carbonates, les hydrogénocarbonates, les sulfates, les hydrogénophosphates, les phosphates.

Les sels organiques utilisables selon l'invention sont par exemple les sels de triéthanolamine, mono-éthanolamine, éthanolamine, hexadécylamine et N,N,N',N'-tétrakis-(hydroxy-propyl-2) éthylène diamine ainsi que ceux des acides organiques comme les citrates, les lactates, les glycolates, les gluconates, les acétates, les propionates, les fumarates, les oxalates et les tartrates.

Comme solvates possibles des composés de formule (I), on peut citer les hydrates, les alcoolates ou les hydroalcoolates.

Selon l'invention, les composés de formule (I) sont sous forme isolée, c'est-à-dire non polymérique.

"Au moins un" selon l'invention signifie un ou plusieurs (2, 3 ou plus). En particulier, la composition peut contenir un ou plusieurs composés de formule (I). Ce ou ces composés peuvent être des isomères cis ou trans ou Z ou E ou un mélange d'isomères cis/trans ou Z/E. Ils peuvent aussi être sous forme tautomère. Ce ou ces composés peuvent être des énantiomères et/ou des diastéréoisomères ou un mélange de ces isomères, en particulier un mélange racémique.

Par "radical alkyle" on entend au sens de l'invention un radical hydrocarboné qui peut être linéaire ou ramifié et saturé ou insaturé. En particulier, le radical alkyle comporte de 1 à 20 atomes de carbone, de préférence de 1 à 10. Comme exemple de radical alkyle utilisable dans l'invention, on peut citer les radicaux méthyle, éthyle, isopropyle, n-butyle, *ter*-butyle, n-hexyle, éthyle-2-hexyle, éthylène, propylène.

Comme atome d'halogène utilisable dans l'invention, on peut citer les atomes de chlore, de fluor ou de brome, et mieux les atomes de chlore et de fluor.

Comme cycles saturés utilisables dans l'invention on peut citer le radical cyclopropyle, cyclobutyle, cyclopentyle, cyclohexyle ou cycloheptyle, adamantyle..

Comme cycles insaturés, on peut citer le radical cyclohexényle ou phényle. Comme cycles hydrocarbonés accolés, utilisables dans l'invention, on peut citer le radical naphtyle, azulényle.

Comme cycles accolés de nature différente utilisables dans l'invention, on peut citer les cycles benzofuranne, benzothiophène, benzothiazole, indole, benzimidazole, quinoline, isoquinoline, quinazoline, chromène, carbazole, fluorène.

Comme exemple de cycle à fonction carbonyle ou thiocarbonyle utilisable dans l'invention, on peut citer les cycles suivants :

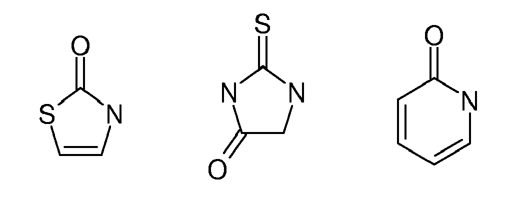

Comme exemple d'hétérocycles utilisables dans l'invention, on peut citer indépendamment les cycles azétidine, pyrrole, dihydropyrrole, pyrrolidine, furanne, dihydrofuranne, tétrahydrofuranne, thiophène, dihydrothiophène, tétrahydrothiophène, imidazole, dihydro-imidazole, imidazolidine, thiazole, dihydrothiazole, thiazolidine, pyrazole, dihydropyrazole, pyrazolidine, oxazole, dihydro-oxazole, oxazolidine, isoxazole, dihydro-isoxazole, isoxazolidine, isothiazole, dihydro-isothiazole, isothiazolidine, triazole, dihydrotriazole, triazolidine, oxadiazole, dihydro-oxadiazole, oxadiazolidine, thiadiazole, dihydrothiadiazole, thiadiazolidine, tétrazole, pyridine, dihydropyridine, tétrahydropyridine, pipéridine, pyranne, dihydropyranne, tétrahydropyranne, pyrimidine, dihydropyrimidine, tétrahydro-pyrimidine, pipérazine, pyridazine, pyrazine, triazine, morpholine, azépine, diazépine. De préférence, on utilise les cycles pyrrole, pyrrolidine, imidazole, furanne, thiophène, oxazole, thiazole, isoxazole, isothiazole, oxadiazole, pyrazole, tétrazole, pyridine, pyrimidine, triazole, pyrazine, pyridazine, pipéridine, pipérazine, morpholine.

Parmi les composés de formule (I) conformes à l'invention, on peut citer plus particulièrement ceux choisis dans la liste de composés suivants :

| **Composé** | **Structure** |
|---|---|
| 1 | 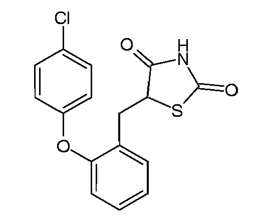 |
| 2 | 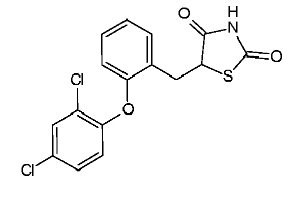 |
| 3 | 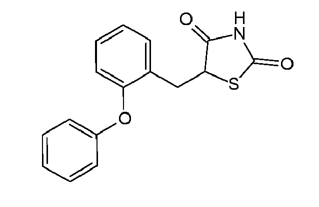 |
| 4 | 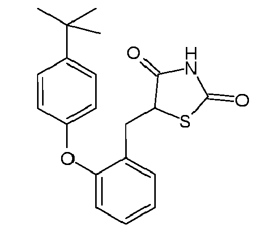 |
| 5 | 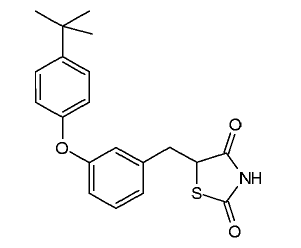 |
| 6 | 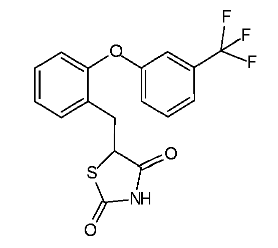 |

Les composés de formule (I) sont choisis de préférence parmi ceux pour lesquelles les conditions suivantes sont remplies
- A₁, A₂, A₃, A₄ désignent simultanément hydrogène ;
- A₅ désigne un groupe Z₁
- Z₁ désigne un groupe OZ₂ dans lequel Z₂ désigne un groupe phényle éventuellement substitué par un ou plusieurs groupes adamantyle, CF₃, halogène, alkyle en C₁-C₂₀ linéaire ou ramifié, saturé ou insaturé.

Le composé plus particulièrement préféré est le composé 5-[2-(2-(4-tert-butylphenoxy)benzyl)]-1,3-thiazolidine-2,4-dione de structure :

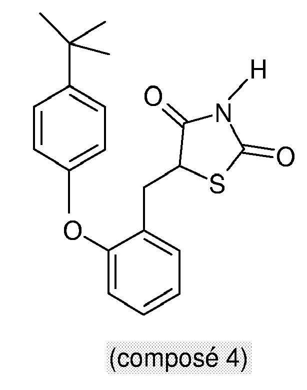

Les composés de formule (I) conformes à l'invention peuvent être obtenus selon une voie de synthèse telle que décrite dans le schéma réactionnel défini ci-dessus. La voie de synthèse utilisée consiste en une substitution nucléophile aromatique, une condensation de Knoevenagel, puis une réduction de la double liaison :

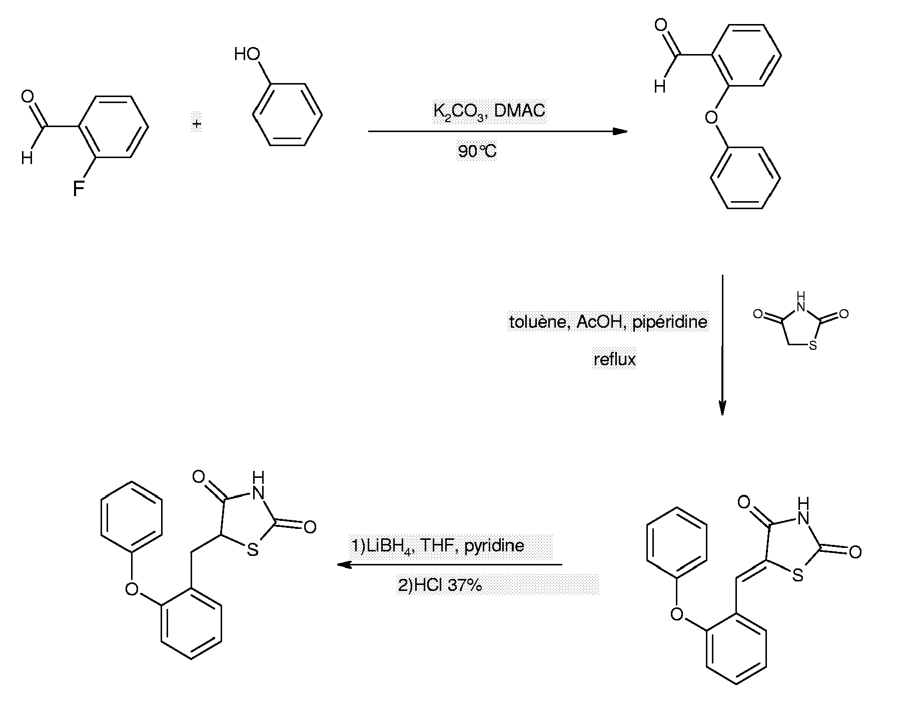

La quantité efficace de composé de formule (I) (salifié ou non, solvaté ou non) correspond à la quantité nécessaire de composé pour obtenir le résultat désiré (à savoir induire, stimuler la pigmentation kératiniques et notamment des cheveux et des cils et/ou réduire leur dépigmentation et/ou leur blanchiment). L'homme du métier est donc en mesure d'évaluer cette quantité efficace qui dépend de la nature du composé utilisé, de la personne à laquelle on l'applique, et du temps de cette application.

Dans la suite du texte, et sauf indication contraire, les quantités des différents ingrédients de la composition sont données en pourcentage en poids par rapport au poids total de la composition.

Pour donner un ordre de grandeur, selon l'invention, le composé de formule (I) (salifié ou non, solvaté ou non) ou un mélange de composés de formule (I) (salifiés ou non, solvatés ou non) peut être utilisé en une quantité allant de 10⁻³% à 10 % du poids total de la composition et préférentiellement en une quantité allant de 10⁻³% à 5 % et mieux de 10⁻² % à 2 % du poids total de la composition, par exemple de 0,5 % à 2 %.

La composition de l'invention peut être à usage cosmétique ou pharmaceutique. Préférentiellement, la composition de l'invention est à usage cosmétique. Aussi, la composition doit contenir un milieu physiologiquement acceptable non toxique et susceptible d'être appliqué sur la peau, y compris le cuir chevelu et les paupières, et sur les fibres kératiniques d'êtres humains. Par "cosmétique", on entend au sens de l'invention une composition d'aspect, d'odeur et de toucher agréables.

Le composé de formule (I) (salifié ou non, solvaté ou non) peut être utilisé dans une composition qui doit être ingérée, injectée ou appliquée sur la peau ou sur les fibres kératiniques (sur toute zone cutanée ou des fibres à traiter).

Selon l'invention, le composé de formule (I) ou un mélange de composés de formule (I) peut être utilisé par la voie orale en une quantité de 0,1 mg à 300 mg par jour, notamment de 5 mg/j à 10 mg/j.

Une composition préférée de l'invention est une composition à usage cosmétique et en particulier d'application topique sur la peau et les fibres kératiniques, et plus spécialement sur le cuir chevelu, les cheveux et les cils.

Cette composition peut se présenter sous toutes formes galéniques connues adaptées au mode d'utilisation.

Pour une application topique sur la peau ou les fibres kératiniques, la composition peut avoir la forme d'une solution ou suspension aqueuse, alcoolique ou hydro-alcoolique ou d'une suspension ou solution huileuse, d'une émulsion ou dispersion de consistance plus ou moins fluide et notamment liquide ou semi-liquide, obtenue par dispersion d'une phase grasse dans une phase aqueuse (H/E) ou inversement (E/H), d'une émulsion ou dispersion solide (H/E) ou (E/H), d'un gel aqueux, hydro-alcoolique ou huileux plus ou moins fluide ou solide, d'une poudre libre ou compactée à utiliser telle quelle ou à incorporer dans un milieu physiologiquement acceptable, ou encore de microcapsules ou microparticules, de dispersions vésiculaires de type ionique et/ou non ionique.

On peut également envisager une composition sous forme de mousse ou encore sous forme de spray ou d'aérosol comprenant alors un agent propulseur sous pression.

Elle peut ainsi se présenter sous forme d'une lotion, sérum, lait, crème H/E ou E/H, gel, onguent, pommade, poudre, baume, patch, tampon imbibé, pain, mousse.

En particulier la composition à application sur le cuir chevelu ou les cheveux peut se présenter sous forme d'une lotion de soin capillaire, par exemple d'application journalière ou bi-hebdomadaire, d'un shampooing ou d'un après-shampooing capillaire, en particulier d'application bi-hebdomadaire ou hebdomadaire, d'un savon liquide ou solide de nettoyage du cuir chevelu d'application journalière, d'un produit de mise en forme de la coiffure (laque, produit pour mise en pli, gel coiffant), d'un masque traitant, d'une crème ou d'un gel moussant de nettoyage des cheveux. Elle peut encore se présenter sous forme de teinture ou de mascara capillaire à appliquer au pinceau ou au peigne.

Par ailleurs, pour une application sur les cils ou les poils, la composition à laquelle s'applique l'invention peut se présenter sous forme d'un mascara, pigmenté ou non, à appliquer à la brosse sur les cils ou encore sur les poils de barbe ou de moustache.

Pour une composition à usage par injection, la composition peut se présenter sous forme de lotion aqueuse ou de suspension huileuse. Pour un usage par voie orale, la composition peut se présenter sous forme de capsules, de granulés, de sirops buvables ou de comprimés.

Selon un mode de réalisation particulier, la composition selon l'invention se présente sous forme de crème ou lotion capillaire, de shampooing ou d'après-shampooing capillaire, de mascara capillaire ou pour cils.

Les quantités des différents constituants du milieu physiologique de la composition selon l'invention sont celles généralement utilisées dans les domaines considérés. En outre, ces compositions sont préparées selon les méthodes usuelles.

Lorsque la composition est une émulsion, la proportion de la phase grasse peut aller de 2 % à 80 % en poids, et de préférence de 5 % à 50 % en poids par rapport au poids total de la composition. La phase aqueuse est ajustée en fonction de la teneur en phase grasse et en composé(s) (I) ainsi que de celle des éventuels ingrédients additionnels, pour obtenir 100% en poids. En pratique la phase aqueuse représente de 5 % à 99,9% en poids.

La phase grasse peut contenir des composés gras ou huileux, liquides à température ambiante (25°C) et pression atmosphérique (760 mm de Hg), généralement appelés huiles. Ces huiles peuvent être compatibles ou non entre elles et former une phase grasse liquide macroscopiquement homogène ou un système bi- ou triphasique.

La phase grasse peut, en plus des huiles, contenir des cires, des gommes, des polymères lipophiles, des produits "pâteux" ou visqueux contenant des parties solides et des parties liquides.

La phase aqueuse contient de l'eau et éventuellement un ingrédient miscible en toute proportion à l'eau comme les alcools inférieurs en C₁ à C₈ tel que l'éthanol, l'isopropanol, les polyols comme le propylène glycol, le glycérol, le sorbitol ou encore l'acétone ou l'éther.

Pour une composition sous forme d'émulsion, la composition peut contenir un ou plusieurs émulsionnants associés éventuellement à un ou plusieurs co-émulsionnants utilisés pour l'obtention d'une composition sous forme d'émulsion, ces émulsionnants et co-émulsionnants sont ceux généralement utilisés dans les domaines cosmétique et pharmaceutique. Leur nature est, en outre, fonction du sens de l'émulsion. En pratique, l'émulsionnant et éventuellement le co-émulsionnant sont présents, dans la composition, en une proportion allant de 0,1 % à 30 % en poids, de préférence de 0,5 % à 20 % en poids et mieux de 1 à 8 %. L'émulsion peut, en outre, contenir des microcapsules ou microparticules, des dispersions vésiculaires et notamment des vésicules lipidiques et telles que des liposomes.

Lorsque la composition est sous forme d'une solution ou d'un gel huileux, la phase grasse peut représenter plus de 90 % du poids total de la composition.

Avantageusement, pour une application capillaire, la composition de l'invention est une solution ou suspension aqueuse, alcoolique ou hydro-alcoolique et mieux une solution ou suspension eau/éthanol. La fraction alcoolique peut représenter de 5% à 99,9% et mieux de 8% à 80%.

Pour une application mascara, la composition de l'invention est notamment sous forme d'une dispersion de cire-dans-eau ou de cire-dans-huile, d'une huile gélifiée, d'un gel aqueux, pigmenté ou non.

La composition de l'invention peut comprendre, en outre, d'autres ingrédients additionnels usuellement utilisés dans les domaines concernés, choisis parmi les solvants, les épaississants ou gélifiants de phase aqueuse ou de phase huileuse, les matières colorantes solubles dans le milieu de la composition, les particules solides du type charges ou pigments, les antioxydants, les séquestrants, les conservateurs, les parfums, les électrolytes, les neutralisants, les polymères filmogènes, les agents bloqueurs d'U.V. comme les filtres solaires, les actifs cosmétiques et pharmaceutiques à action bénéfique pour la peau ou les fibres kératiniques, autres que les composés de formule (I), leurs mélanges. Ces additifs peuvent être présents dans la composition selon les quantités généralement utilisées dans le domaine cosmétique et dermatologique et notamment à raison de 0,01 % à 50% du poids total de la composition et mieux de 0,1 % à 20 % et par exemple de 0,1 % à 10 %. Ces additifs selon leur nature, peuvent être introduits dans la phase grasse, dans la phase aqueuse et/ou dans les vésicules lipidiques et notamment des liposomes.

Bien entendu l'homme du métier veillera à choisir les éventuels ingrédients additionnels et/ou leur quantité de telle manière que les propriétés avantageuses de la composition selon l'invention, à savoir l'inhibition de la 15-PGDH et notamment l'augmentation de la densité des fibres kératiniques et/ou la réduction de leur blanchiment, ne soient pas ou substantiellement pas, altérées par l'adjonction envisagée.

Comme solvants utilisables dans l'invention, on peut citer les alcools inférieurs en C₂ à C₈ comme l'éthanol, l'isopropanol, le propylène glycol et certaines huiles cosmétiques légères comme les alcanes en C₆ à C₁₆.

Comme huiles utilisables dans l'invention, on peut citer les huiles d'origine minérale (huile de vaseline, isoparaffine hydrogénée), les huiles d'origine végétale (fraction liquide du beurre de karité, huile de tournesol, d'abricot, alcool ou acide gras), les huiles d'origine animale (perhydrosqualène), les huiles de synthèse (ester d'acide gras, huile de Purcellin), les huiles siliconées (polydiméthylsiloxane linéaire ou cyclique, phényltriméthicone) et les huiles fluorées (perfluoropolyéthers). Comme cires, on peut citer les cires siliconées, les cires d'abeille, de riz, de candellila, de carnauba ou paraffine, de polyéthylène.

Comme émulsionnants utilisables dans l'invention, on peut citer par exemple le stéarate ou laurate de glycérol, les stéarates ou oléates de sorbitol, les alkyl diméthiconecopolyol (avec alkyle ≥ 8) et leurs mélanges pour une émulsion E/H. On peut aussi utiliser le monostéarate ou monolaurate de polyéthylène glycol, le stéarate ou oléate de sorbitol polyoxyéthyléné, les diméthiconecopolyols et leurs mélanges pour une émulsion H/E.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les Bentones, les sels métalliques d'acides gras comme les stéarates d'aluminium, la silice traitée hydrophobe, l'éthylcellulose, leurs mélanges.

Comme actif cosmétique ou pharmaceutique, autre que le composé de formule (I) utilisable dans l'invention, on peut citer les actifs hydrophiles comme les protéines ou hydrolysats de protéine, les acides aminés, les polyols, l'urée, l'allantoïne, les sucres et les dérivés de sucre, les vitamines hydrosolubles, les extraits végétaux (ceux d'Iridacées ou de soja) et les hydroxy-acides comme les acides de fruits ou l'acide salicylique ; et les actifs lipophiles comme le rétinol (vitamine A) et ses dérivés notamment ester (palmitate de rétinol), le tocophérol (vitamine E) et ses dérivés notamment ester (acétate de tocophérol), les acides gras essentiels, les céramides, les huiles essentielles, les dérivés de l'acide salicylique comme le n-octanoyl-5 salicylique, les esters des hydroxy-acides, les phospholipides comme la lécithine, leurs mélanges.

Avantageusement, la composition selon l'invention comprend en plus au moins une prostaglandine ou un dérivé de prostaglandine comme par exemple les prostaglandines de la série 2 dont notamment PGF2-α et PGE2 sous forme saline ou ester (exemple les isopropyl esters), leurs dérivés comme le 16,16 diméthyl PGE2, le 17 phényl PGE2, le 16,16 diméthyl PGF2-α, le 17 phényl PGF2-α les prostaglandines de la série 1 comme le 11 déoxy prostaglandine E1, le 1 déoxy prostaglandine E1 sous forme saline ou ester, leurs analogues notamment le latanoprost, l'acide (5E)-7-{(1 R,2R,3R,5S)-3,5-dihydroxy-2-[(3R)-3-hydroxy-5-phénylpentyl]cyclopentyl}hept-5-énoique, le viprostol, le bimatoprost, le cloprosténol le travoprost, le fluprosténol, le cloprosténol, le butaprost, l'unoprostone, le misoprostol, leurs sels ou leurs esters.

On peut également envisager que la composition comprenant au moins le composé de formule (I), salifié ou non, solvaté ou non, soit encapsulé notamment sous forme de liposome, telle que notamment décrite dans le document WO 94/22468. Ainsi, le composé encapsulé peut être délivré sélectivement au niveau du follicule pileux.

La composition selon l'invention peut être appliquée sur les zones de la peau à traiter et en particulier sur les zones alopéciques du cuir chevelu et des cheveux d'un individu, ou uniquement sur les cheveux blancs, et éventuellement laissée en contact plusieurs heures et éventuellement rincée.

On peut, par exemple, appliquer la composition contenant une quantité efficace d'un composé de formule (I), salifié ou non, solvaté ou non, le soir, garder celle-ci au contact toute la nuit et éventuellement effectuer un shampooing le matin. Ces applications peuvent être renouvelées quotidiennement pendant un ou plusieurs mois suivant les individus.

Avantageusement, dans le procédé selon l'invention, on applique sur les zones à soigner ou traiter du cuir chevelu et/ou des cheveux entre 5 µL et 10 ml d'une solution ou composition telle que définie précédemment, comprenant de 0,001% à 5 % d'inhibiteur de la 15-PGDH.

On va maintenant donner à titre d'illustration des exemples de réalisation de l'invention qui ne sauraient limiter en aucune façon sa portée.

### Exemple 1

### Synthèse du composé 5-(2-phenoxybenzyl)-1,3-thiazolidine-2,4-dione de formule (I) (A₁ = A₂ = A₃ = A₄ = H ; A₅ = OZ₂ ; Z₂ est phényle)

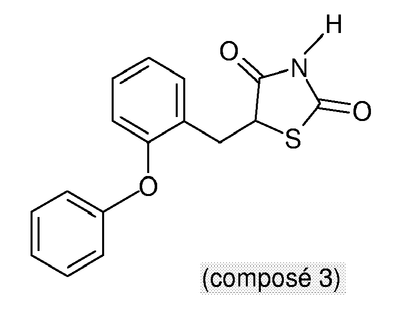

### 1 ère étape, Synthèse de 2-phénoxybenzaldéhyde :

Dans un tricol de 1000 mL sous argon, sont introduits le phénol (50g ; 0.531 mole), le carbonate de potassium (80.7g ; 0.584 mole) puis le diméthylacétamide (300 mL). Le 2-fluorobenzaldéhyde (55.9 mL ; 0.531 mole) est alors additionné. Le milieu réactionnel est hétérogène légèrement jaune. Ce mélange est chauffé à 120°C pendant 6 heures. Après retour à température ambiante, le milieu est dilué avec de l'eau et extrait deux fois avec du dichlorométhane. La phase organique est lavée avec une solution d'hydrogénocarbonate de sodium à 5% puis avec de l'eau et enfin avec une solution saturée en chlorure de sodium. Elle est ensuite séchée sur du sulfate de sodium puis filtrée et concentrée au maximum. L'huile jaune-marron obtenue est distillée sous vide en chauffant. 70g d'huile légèrement jaune sont obtenus (Rdt : 67%).

### 2ème étape, Synthèse de (5Z)-5-(2-phenoxybenzylidene)-1,3-thiazolidine-2,4-dione :

Dans un ballon de 1000 mL surmonté d'un Dean-Stark et d'un réfrigérant, est dilué le 2-phénoxybenzaldéhyde (70g ; 0.353 mole) dans 500 mL de toluène. Sont alors ajoutés la 2,4-thiazolidinedione (41.4g ; 0.3533 mole), la pipéridine (10 mL) et l'acide acétique (10 mL). Le milieu réactionnel est porté à reflux pendant 4 heures. Après retour à température ambiante, il y a formation d'un précipité qui est filtré puis rincé plusieurs fois avec du toluène. Le produit obtenu est séché sous vide à 50°C pour donner 100g d'un solide jaune (Rdt : 96%).

### 3ème étape, synthèse de 5-(2-phenoxybenzyl)-1,3-thiazolidine-2,4-dione :

Dans un tricol de 50mL sous argon, est dissoute la (5Z)-5-(2-phénoxybenzylidène)-1,3-thiazolidine-2,4-dione (1g ; 3.3 10-3 mole) dans le tétrahydrofurane anhydre (3 mL) et dans la pyridine (3 mL). Le borohydrure de lithium 2M (3.7 mL; 7.4 10-3 mole) est additionné lentement, puis le milieu réactionnel est chauffé à 65°C pendant 5 heures. Après retour à 20°C, le milieu réactionnel est versé dans une solution d'acide chlorhydrique refroidie à 5°C. Le mélange est alors extrait trois fois avec de l'acétate d'éthyle. La phase organique est lavée avec une solution saturée en chlorure de sodium. Elle est ensuite séchée sur du sulfate de sodium puis filtrée et concentrée au maximum. On obtient 0,67 g d'une huile qui cristallise (Rdt : 67%). Le produit obtenu est jaune clair.

Analyses
RMN 1 H (DMSO) : 3,09 (dd, 1 H) ; 3,5 (dd, 1 H) ; 4,9 (m, 1H) ; 6,83 (d, 1H) ; 6,98 (dd, 2H) ; 7,12 (m, 1 H) ; 7,15 (m, 1 H) ; 7,28 (m, 1 H) ; 7,34 (dd, 1H) ; 7,4 (m, 2H) ; 12,05 (s, 1 H)
Analyse élémentaire : C16H13NO3S
Théorique % : C 64,2 ; H 4,4 ; N 4,7 ; O : 16,0 ; S 10,7
Mesurée % : C 64,28 ; H 4,51 ; N 4,6 ; O : 16,19 ; S 10,61
Point de fusion : 84-85 °C

### Exemple 2

### Synthèse du composé 5-[3-(2-(4-tert-butylphenoxy)benzyl)]-1,3-thiazolidine-2,4-dione de formule (I) ( A₁ = A₂ = A₃ = A₄ = H ; A₅ = OZ₂ ; Z₂ *est phényle substitué par un groupe tert-butyle)

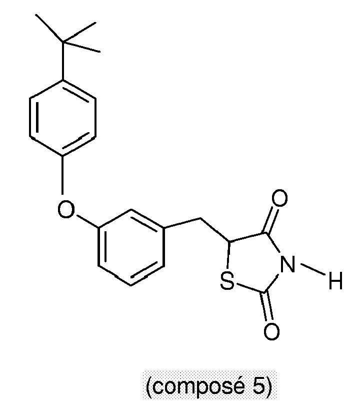

### Première étape: Synthèse de (5Z)-5-[3-(4-tert-butylphenoxy)benzylidene]-1,3-thiazolidine-2,4-dione

Dans un ballon de 250 mL surmonté d'un Dean-Stark et d'un réfrigérant, est dilué le 3-[4-(tert-butyl)phénoxybenzaldéhyde (10g ; 39.3 10⁻³ mole) dans 120 mL de toluène puis sont additionnés la 2,4-thiazolidinedione (4.6g ; 39.9 10⁻³ mole), la pipéridine (1 mL) et l'acide acétique (1 mL). Le milieu réactionnel est porté à reflux pendant 4 heures. Après retour à température ambiante, il y a formation d'un précipité qui est filtré puis rincé plusieurs fois avec du toluène. Le solide jaune clair obtenu est séché sous vide à 50°C pour donner 8.54g de produit (Rdt : 62%).

### Deuxième étape : Synthèse de 5-[3-(4-tert-butylphenoxy)benzyl]-1,3-thiazolidine-2,4-dione

Dans un tricol de 50mL sous argon, est dissout le (5Z)-5-[3-(4-tert-butylphénoxy)benzylidène]-1,3-thiazolidine-2,4-dione (3g ; 8.5 10-3 mole) dans le tétrahydrofurane anhydre (9 mL) et dans la pyridine (9 mL). Le borohydrure de lithium 2M (9.4 mL ; 18.7 10-3 mole) est additionné lentement, puis le milieu réactionnel chauffé à 65°C pendant 6 heures. Après retour à 20°C, le milieu réactionnel est versé lentement dans une solution d'acide chlorhydrique refroidie à 10°C. Ce mélange est extrait trois fois avec de l'acétate d'éthyle et la phase organique lavée avec une solution saturée en chlorure de sodium. Elle est ensuite séchée sur du sulfate de sodium puis filtrée et concentrée au maximum. Le produit brut est dilué dans du dichlorométhane auquel est ajouté du noir végétal. Ce mélange est agité pendant 3 heures, puis filtré sur de la célite et concentré au maximum. 1.85g d'une pâte jaune est obtenue (Rdt : 62%).

Analyses
RMN ¹H (DMSO) : 1,28 (s, 9H) ; 3,13 (dd, 1 H) ; 3,37 (dd, 1 H) ; 4,91 (dd, 1 H) ; 6,86 (dd, 1 H) ; 6,91 (m, 2H) ; 6,93 (m, 1H) ; 7 (de, 1H) ; 7,31 (t, 1 H) ; 7,39 (m, 2H) ; 12 (se, 1 H)
Analyse élémentaire : C₂₀H₂₁NO₃S
Théorique % : C 67.6 ; H 6.0 ; N 3.9 ; O 13.5 ; S 9.0
Mesurée % : C67.31 ; H 6.03 ; N 4.04 ; O 13.69 ; S 8.79

### EXEMPLE 3 : Mise en évidence des propriétés inhibitrices spécifiques de la 15-PGDH des composés de formule (I)

### Test sur 15-PGDH de type 1 :

L'enzyme 15-PGDH est obtenue comme décrit dans la demande FR 02/05067 déposée au nom de L'Oréal, en suspension dans un milieu adapté à une concentration de 0,3 mg/mL puis bloquée à - 80°C. Pour les besoins du test, cette suspension est décongelée et stockée dans de la glace.

Par ailleurs on prépare un tampon Tris 100 mM, pH = 7,4, contenant 0,1 mM de dithiothréitol (D5545, Sigma-Aldrich, L'isle D'Abeau Chesne, BP 701, 38297, Saint Quentin Fallavier), 1,5 mM de β-NAD (N6522, Sigma-Aldrich, L'isle D'Abeau Chesne, BP 701, 38297, Saint Quentin Fallavier), 50 µM de Prostaglandine E₂ (P4172, Sigma-Aldrich, L'isle D'Abeau Chesne, BP 701, 38297, Saint Quentin Fallavier).

Dans la cuve d'un spectrophotomètre (Perkin-Elmer, Lambda 2) thermostaté à 37°C, dont la longueur d'onde de mesure est réglée à 340 nm, sont introduits 0,965 ml de ce tampon (préalablement porté à 37°C). 0,035 mL de suspension enzymatique à 37°C sont introduits dans la cuve concomitamment à l'enregistrement (correspondant à une augmentation de la densité optique à 340 nm). La vitesse maximale de réaction est relevée.

Les valeurs essais (contenant les composés de formule (I)) sont comparées à la valeur témoin (sans composé de formule (I)) ; les résultats indiqués représentent soit le pourcentage d'inhibition de l'activité enzymatique de 15-PGDH pour une concentration donnée de composé de formule (I), soit la concentration à laquelle le composé de formule (I) réduit de 50% l'activité enzymatique de 15-PGDH, à savoir IC_{50dh}.

Les résultats sont donnés dans le tableau ci-après.

| **Composé** | **Structure** | **IC_{50dh} ou% inhibition à 42 µM** |
|---|---|---|
| 1 | 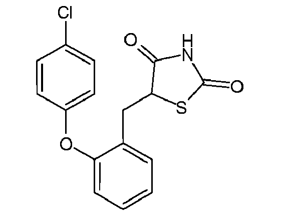 | 2,2 µM |
| 2 | 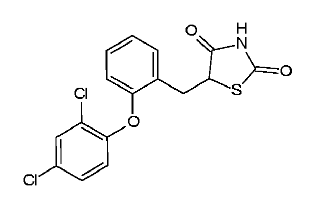 | 1,21 µM |
| 3 | 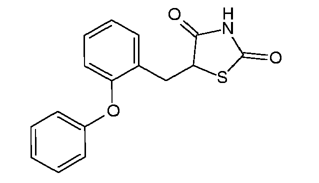 | 2,75 µM |
| 4 | 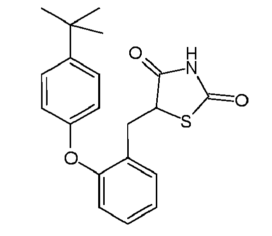 | 0,4 µM |
| 5 | 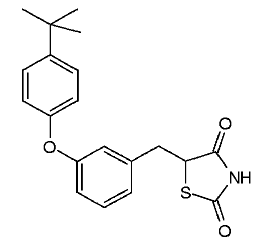 | 0,58 µM |
| 6 | 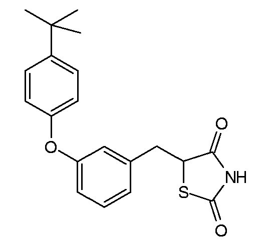 | 91% d'inhibition à 42 µM |

De ces tableaux, il ressort clairement que les composés de formule (I) ou (II) sont des inhibiteurs de 15-PGDH de type 1.

Les compositions ci-après sont obtenues par les techniques habituelles couramment utilisées dans le domaine cosmétique ou pharmaceutique.

### EXEMPLE 4 : Lotion capillaire

| | | |
|---|---|---|
| 5-[2-(4-tert-butylphenoxy)benzyl]-1,3-thiazolidine-2,4-dione | | |
| (composé 4) | | 1,00 g |
| Propylène glycol | | 30,00 g |
| Alcool éthylique | | 40,00 g |
| Eau | qsp | 100,00 g |

On applique cette lotion sur le cuir chevelu, une à deux fois par jour, à raison d'1 ml par application, en massant légèrement le cuir chevelu pour faire pénétrer l'actif. La chevelure est ensuite séchée à l'air libre. Cette lotion permet de prévenir et/ou diminuer la canitie des cheveux.

### EXEMPLE 5 : Lotion capillaire

| | | |
|---|---|---|
| 5-[2-(4-tert-butylphenoxy)benzyl]-1,3-thiazolidine-2,4-dione | | |
| (composé 4) | | 1,00 g |
| Propylène glycol | | 30,00 g |
| Alcool éthylique | | 40,00 g |
| - Eau | qsp | 100,00 g |

On applique cette lotion sur le cuir chevelu, une à deux fois par jour, à raison d'1 ml par application, en massant légèrement le cuir chevelu pour faire pénétrer l'actif. La chevelure est ensuite séchée à l'air libre. Cette lotion permet de prévenir et/ou diminuer la canitie des cheveux.

### EXEMPLE 6 : Mascara cire/eau

| | | |
|---|---|---|
| Cire d'abeilles | | 6,00 % |
| Cire de paraffine | | 13,00 % |
| Huile de jojoba hydrogénée | | 2,00 % |
| Polymère filmogène hydrosoluble | | 3,00 % |
| Stéarate de triéthanolamine | | 8,00 % |
| 5-[2-(4-tert-butylphenoxy)benzyl]-1,3-thiazolidine-2,4-dione | | |
| (compose 4) | | 1,00 % |
| Pigment noir | | 5,00 % |
| Conservateur | qs | |
| Eau | qsp | 100,00 % |

Ce mascara s'applique sur les cils comme un mascara classique avec une brosse à mascara. Il permet de prévenir et/ou diminuer la canitie des cils.

### EXEMPLE 7 : Lotion capillaire

| | | |
|---|---|---|
| 5-[2-(4-tert-butylphenoxy)benzyl]-1,3-thiazolidine-2,4-dione | | |
| (composé 4) | | 0,10 g |
| Latanoprost | | 0,10 g |
| Propylène glycol | | 30,00 g |
| Alcool éthylique | | 40,00 g |
| Eau | qsp | 100,00 g |

On applique cette lotion sur le cuir chevelu, une à deux fois par jour, à raison d'1 ml par application, en massant légèrement le cuir chevelu pour faire pénétrer l'actif. La chevelure est ensuite séchée à l'air libre. Cette lotion permet de prévenir et/ou diminuer la canitie des cheveux.

### EXEMPLE 8 : Lotion capillaire

| | | |
|---|---|---|
| Acide (5E)-7-{(1R,2R,3R,5S)-3,5-dihydroxy-2-[(3R)-3- | | |
| hydroxy-5-phényl pentyl]cyclopentyl}hept-5-énoique | | 0,10 g |
| 5-[2-(4-tert-butylphenoxy)benzyl] | | |
| -1,3-thiazolidine-2,4-dione | | 0,10 g |
| Propylène glycol | | 30,00 g |
| Alcool éthylique | | 40,00 g |
| Eau | qsp | 100,00 g |

On applique cette lotion sur le cuir chevelu, une à deux fois par jour, à raison d'1 ml par application, en massant légèrement le cuir chevelu pour faire pénétrer l'actif. La chevelure est ensuite séchée à l'air libre. Cette lotion permet de prévenir et/ou diminuer la canitie des cheveux.

## Revendications

1. Utilisation cosmétique d'au moins un composé benzyl-1,3-thiazolidine-2,4-dione répondant à la formule (I) suivante ou correspondant à l'un de ses sels et/ou de ses solvates : 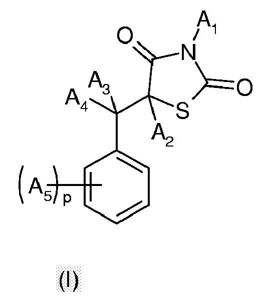
a) A₁ désigne :
1) un atome d'hydrogène ;
2) un groupe alkyle en C₁-C₂₀ linéaire ou ramifié, saturé ou insaturé éventuellement substitué par un ou plusieurs groupes Z₁ ;
b) A₂, A₃, A₄ et A₅ désignent indépendamment :
1) un atome d'hydrogène ;
2) un groupe Z₁ ;
c) p est compris entre 0 et 5 inclus et dans le cas où n>1, les substituants A₅ peuvent être identiques ou différents ;
d) Z₁ désigne
1) un halogène comme F, CI, Br,
2) un groupe choisi parmi CF₃, OCF₃, CN, NO₂, OZ₂, OCOZ₂, OCONZ₂Z'₂, SZ₂, SCOZ₂, SCONZ2Z'₂, SCSOZ₂, SCSNZ₂Z'₂, NZ₂Z'₂, NZ₂COZ'₂, NZ₂CONZ'₂Z''₂, NZ₂C(=NZ'₂)NZ''₂Z'''₂, NZ₂SO₂Z'₂, COZ₂, COOH, CONZ₂Z'₂, CSZ₂, CSNZ₂Z'₂ SO₃Z, SO₂NZ₂Z'₂, SO₂Z₂, SiZ₂Z'₂Z''₂, Si(OZ₂)(OZ'₂)OZ''₂ ;
3) un groupe alkyle en C₁-C₂₀, linéaire ou ramifié, saturé ou insaturé,
e) les radicaux Z₂, Z'₂, Z''₂ et Z'''₂ indépendamment désignent :
1) un atome d'hydrogène ;
2) un groupe alkyle en C₁-C₂₀ linéaire ou ramifié, saturé ou insaturé, éventuellement substitués par un ou plusieurs groupes Z₃ ;
3) un cycle de 4 à 15 atomes, saturés ou insaturés, contenant éventuellement au moins un hétéroatome parmi O, N, S, éventuellement accolé à un autre cycle, ces cycles étant éventuellement substitués par un ou plusieurs groupes Z₃. Ce cycle est avantageusement un phényle ;
f) le radical Z₃ représente :
1) un groupe Z₄ ;
2) un groupe alkyle en C₁-C₂₀ linéaire ou ramifié, saturé ou insaturé, éventuellement substitué par un ou plusieurs groupes Z₄ ;
3) un cycle de 4 à 15 atomes, saturés ou insaturés, contenant éventuellement au moins un hétéroatome parmi O, N, S, éventuellement accolé à un autre cycle, ces cycles étant éventuellement substitués par un ou plusieurs groupes Z₄ ;
g) Z₄ représente :
1) un halogène comme F, CI, Br,
2) un des groupes CF₃, OCF₃, CN, NO₂, OZ₅, OCOZ₅, OCONZ₅Z'₅, SZ5, SCOZ₅, SCONZ₅Z'₅, SCSOZ₅, SCSNZ₅Z'₅, NZ₅Z'₅, NZ₅COZ'₅, NZ₅CONZ'₅Z"₅, NZ₅C(=NZ'₅)NZ"₅Z'''₅, NZ₅SO₅Z'₅, COZ₅, COOZ₅, CONZ₅Z'₅,CSZ₅, CSNZ₅Z'₅, SO₃Z, SO₂NZ₅Z'₅, SO₂Z5, SiZ₅Z'₅Z"₅, Si(OZ₅)(OZ'₅)OZ"₅;
h) les radicaux Z₅, Z'₅, Z"₅ et Z"'₅ indépendamment représentent
1) un atome d'hydrogène
2) un groupe alkyle en C₁-C₂₀ linéaire ou ramifié, saturé ou insaturé,
3) un cycle de 4 à 15 atomes, saturés ou insaturés, contenant éventuellement au moins un hétéroatome parmi O, N, S, éventuellement accolé à un autre cycle, ces cycles étant éventuellement substitués par un ou plusieurs groupes CF₃, halogène, alkyle en C₁-C₂₀ linéaire ou ramifié, saturé ou insaturé.
sous réserve que lorsque A₅ est situé en para du groupe CA₃A₄ :
(i) Lorsque Z₁ désigne OZ₂ ou COZ₂ alors Z₂ désigne un cycle tel que défini précédemment ;
(ii) A₅ n'est pas un phényle ;
(iii) Lorsque Z₁ désigne NZ₂Z'₂ alors Z₂ désigne un groupe alkyle tel que défini précédemment
comme agent pour favoriser et/ou induire et/ou stimuler la pigmentation des matières kératiniques humaines et/ou comme agent pour prévenir et/ou limiter la dépigmentation et/ou le blanchiment des matières kératiniques.

2. Utilisation selon la revendication 1, **caractérisée en ce que** les composés de formule (I) sont choisis parmi ceux pour lesquelles les conditions suivantes sont remplies
- A₁, A₂ ,A₃ et A₄ désignent simultanément hydrogène ;
- A₅ désigne un groupe Z₁
- Z₁ désigne un groupe OZ₂ dans lequel Z₂ désigne un groupe phényle éventuellement substitué par un ou plusieurs groupes adamantyle, CF₃, halogène, alkyle en C₁-C₂₀ linéaire ou ramifié, saturé ou insaturé

3. Utilisation selon la revendication 1 ou 2, **caractérisée en ce que** les composés de formule (I) sont choisis dans la liste des composés suivants :
| **Composé** | **Structure** |
|---|---|
| 1 | 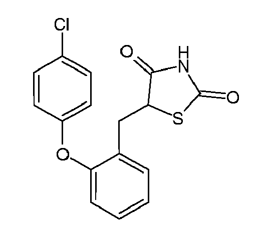 |
| 2 | 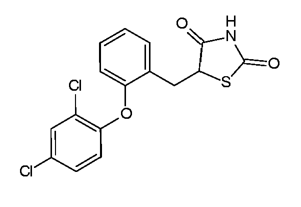 |
| 3 | 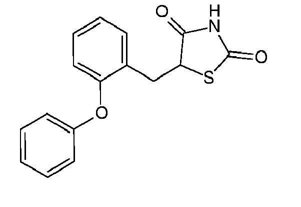 |
| 4 | 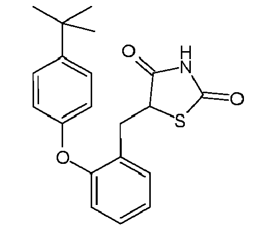 |
| 5 | 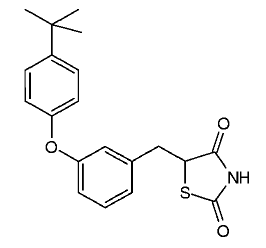 |
| 6 | 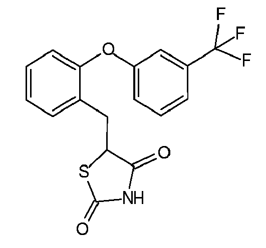 |

4. Utilisation selon la revendication 3, **caractérisée en ce que** le composé de formule (I) est le composé 5-[2-(2-(4-tert-butylphenoxy)benzyl)]-1,3-thiazolidine-2,4-dione de structure :

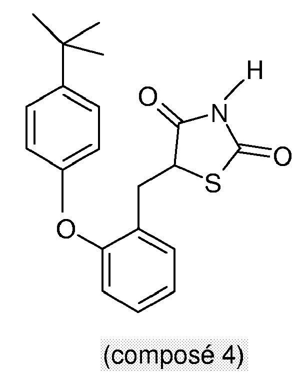

5. Utilisation selon d'une des revendications précédentes, **caractérisée en ce que** le composé de formule (I) ou un mélange de composés de formule (I) est utilisé à une concentration allant de 10-3 à 10 %, de préférence de 10-2 à 2 %, par rapport au poids total de la composition.

6. Utilisation selon l'une des revendications précédentes, **caractérisée en que** la composition est une composition à application topique.

7. Utilisation selon l'une des revendications précédentes, où les matières kératiniques sont choisies parmi les fibres kératiniques humaines et en particulier choisies parmi les cheveux, les poils de barbe, les poils de moustache, les cils et les sourcils humains.

8. Utilisation d'au moins un composé benzyl-1,3-thiazolidine-2,4-dione de formule (I) ou d'un de sels et/ou de ses solvates tel que défini dans l'une quelconque des revendications précédentes comme agent pour prévenir et/ou limiter la canitie des fibres kératiniques humaines et en particulier des cheveux, des poils de barbe, des poils de moustache, des cils et/ou des sourcils humains.

9. Utilisation cosmétique d'au moins un composé benzyl-1,3-thiazolidine-2,4-dione de formule (I) ou d'un de sels et/ou de ses solvates tel que défini dans l'une quelconque des revendications précédentes, dans une composition de soin et/ou de maquillage pour induire et/ou stimuler la pigmentation des matières kératiniques et/ou limiter leur dépigmentation et/ou leur blanchiment.

10. Utilisation selon la revendication 9, où les matières kératiniques sont choisies parmi les fibres kératiniques humaines et en particulier choisies parmi les cheveux, les poils de barbe, les poils de moustache, les cils et les sourcils humains.

11. Utilisation d'au moins un composé benzyl-1,3-thiazolidine-2,4-dione de formule (I) ou d'un de sels et/ou de ses solvates tel que défini dans l'une quelconque des revendications précédentes, dans une composition de soin et/ou de maquillage pour prévenir et/ou limiter la canitie des fibres kératiniques humaines et en particulier choisies parmi les cheveux, les poils de barbe, les poils de moustache, les cils et les sourcils humains.

12. Utilisation d'au moins un composé benzyl-1,3-thiazolidine-2,4-dione de formule (I) ou d'un de sels et/ou de ses solvates tel que défini dans l'une quelconque des revendications précédentes dans la fabrication d'une composition destinée à induire et/ou stimuler la pigmentation des matières kératiniques et/ou limiter leur dépigmentation et/ou leur blanchiment.

13. Utilisation selon la revendication 12, où les matières kératiniques sont choisies parmi les fibres kératiniques humaines et en particulier choisies parmi les cheveux, les poils de barbe, les poils de moustache, les cils et les sourcils humains.

14. Utilisation d'au moins un composé benzyl-1,3-thiazolidine-2,4-dione de formule (I) ou d'un de sels et/ou de ses solvates tel que défini dans l'une quelconque des revendications précédentes, dans la fabrication d'une composition destinée à prévenir et/ou limiter la canitie des fibres kératiniques humaines et en particulier des cheveux, des poils de barbe, des poils de moustache, des cils et/ou des sourcils humains.

15. Procédé cosmétique pour induire et/ou stimuler la pigmentation des matières kératiniques et/ou limiter leur dépigmentation et/ou leur blanchiment, **caractérisé par le fait qu'**il consiste à appliquer sur lesdites matières kératiniques, une quantité efficace d'au moins un composé benzyl-1,3-thiazolidine-2,4-dione de formule (I) ou d'un de sels et/ou de ses solvates tel que défini dans l'une quelconque des revendications précédentes.

16. Procédé selon la revendication 15, où lesdites matières kératiniques sont choisies parmi les fibres kératiniques humaines et en particulier parmi les cheveux, les poils de barbe, les poils de moustache, les cils et les sourcils humains.

17. Procédé cosmétique pour traiter la canitie des fibres kératiniques humaines en particulier les cheveux, les poils de barbe, les poils de moustache, les cils et/ou les sourcils, **caractérisé par le fait qu'**il consiste à appliquer sur lesdites fibres, une quantité efficace d'au moins un composé benzyl-1,3-thiazolidine-2,4-dione de formule (I) ou d'un de sels et/ou de ses solvates tel que défini dans l'une quelconque des revendications précédentes.
